Europäisches Patentamt

European Patent Office

Office européen des brevets

(11) Numéro de publication: **0 199 607**
**B1**

(12) **FASCICULE DE BREVET EUROPEEN**

(45) Date de publication du fascicule du brevet: **05.09.90**

(51) Int. Cl.⁵: **G 01 T 1/29,** G 01 T 1/164, G 01 T 1/202

(21) Numéro de dépôt: **86400344.7**

(22) Date de dépôt: **19.02.86**

(54) **Détecteur multiple pour tomoscintigraphie.**

(30) Priorité: **20.02.85 FR 8502427**

(43) Date de publication de la demande:
**29.10.86 Bulletin 86/44**

(45) Mention de la délivrance du brevet:
**05.09.90 Bulletin 90/36**

(84) Etats contractants désignés:
**AT BE CH DE FR GB IT LI LU NL SE**

(56) Documents cités:
**US-A-4 150 292**

**IEEE TRANSACTIONS ON NUCLEAR SCIENCE, vol. NS-28, no. 2, avril 1981, pages 1736-1740, IEEE, New York, US; C.W. WILLIAMS et al.: "Design of the neuro-ecat: a high-resolution, high efficiency positron tomograph for imaging the adult head or infant torso"**

(73) Titulaire: **MEDICORP RESEARCH LABORATORIES CORPORATION**
**1200 North Federal Highway Suite 200-26**
**Boca Raton Florida 33432 (US)**

(72) Inventeur: **Karcher, Gilles**
**2, Rue Lafayette**
**F-54000 Nancy (FR)**
Inventeur: **Amor, Max**
**9, Square de Liège**
**F-54500 Vandoeuvre (FR)**
Inventeur: **Niddam, Roger**
**43, Allée du Jardin Anglais**
**F-93340 Le Rancy (FR)**
Inventeur: **Villemot, Jean-Pierre**
**Le Trident Rue Cyffle**
**F-54000 Nancy (FR)**

(74) Mandataire: **Laget, Jean-Loup et al**
**Cabinet Pierre Loyer 77, rue Boissière**
**F-75116 Paris (FR)**

Courier Press, Leamington Spa, England.

# EP 0 199 607 B1

(56) Documents cités:

**IEEE TRANSACTIONS ON NUCLEAR SCIENCE, vol. NS-30, no. 1, février 1983, pages 665-670, IEEE, New York, US; S.E. DERENZO et al.: "High resolution positron emission tomography using small bismuth germanate crystals and individual photosensors"**

**JOURNAL OF NUCLEAR MEDICINE, vol. 16, no. 12, décembre 1975, pages 1166-1173, New York, US; S.E. DERENZO et al.: "Analytical study of a high-resolution positron ring detector system for transaxial reconstruction tomography"**

# Description

L'invention concerne un détecteur multiple pour tomoscintigraphie.

Pour étudier un organe, tel que le coeur, le cerveau, ou le foie, en tomoscintigraphie, on utilise généralement une gamma-camera tournante à monocristal sensible à une seule incidence. La gamma-camera présente un champ large d'environ (40 cm), une résolution spatiale et une linéarité excellentes. Cependant, la gamma-camera doit effectuer une mesure pour chaque incidence choisie et, en conséquence, le temps d'acquisition des données permettant la reconstruction des images de l'organe observé est très long.

Il a déjà été proposé également d'utiliser de nombreux cristaux disposés suivant une circonférence autour de l'organe à observer. Ces cristaux son regroupés en sous-ensembles et reliés à des photomultiplicateurs tous identiques. Chacun des cristaux est relié par fibre optique à un nombre défini de photomultiplicateurs, par exemple 2, ce qui permet d'établir un adressage de chacun des cristaux. A cet effet, on attribue à chaque photomultiplicateur le nombre 1 ou 0 suivant l'existence ou non d'une liaison par fibre optique.

Une telle disposition est notamment décrite dans le document US—A—4.150.292 qui prévoit des détecteurs élémentaires constitués chacun de plusieurs détecteurs individuels. Un photomultiplicateur est associé à chaque détecteur individuel. Ce photomultiplicateur est à la fois photomultiplicateur d'énergie et photomultiplicateur de position. Dans ce document sont prévus un circuit électronique de sommation d'énergie et un circuit électronique de détermination de position. Lorsqu'un seuil d'énergie est atteint, alors un circuit de coïncidence émet un signal utilisé pour la reconstruction de l'image tomoscintigraphique.

Par cette méthode, on ne peut également prendre en compte les mesures qu'une par une, ce qui ne présente pas un gain de temps très intéressant. Un but de la présente invention est de s'affranchir de telles méthodes d'examen très longues à mettre en oeuvre en tomoscintigraphie, et de relever en une seule fois toutes les données correspondant à chaque coupe et à chaque incidence, en profitant du fait que l'émission radioactive est isotrope.

A cet effet, le but de l'invention est de prévoir une disposition des cristaux sensibles et un couplage avec des photomultiplicateurs particuliers afin d'en réduire le nombre et d'assurer la prise de plusieurs mesures en même temps.

L'invention a pour objet un détecteur multiple pour tomoscintigraphie, du type à cristal sensible à une seule incidence, comportant des détecteurs élémentaires dont le nombre correspond au produit du nombre d'incidences par le nombre de coupes, pour recevoir simultanément toutes les incidences de toutes les coupes désirées, chaque détecteur élémentaire étant constitué par une barrette comportant un certain nombre de cristaux juxtaposés, et orientée pour recevoir une incidence d'une coupe déterminée, chaque cristal d'une barrette étant relié à deux photomultiplicateurs, caractérisé en ce que lesdits photomultiplicateurs sont de types différents, le premier est un photomultiplicateur d'énergie, le second est un photomultiplicateur de détermination de rang, les photomultiplicateurs du premier type étant en nombre égal au nombre de détecteurs élémentaires et les photomultiplicateurs du second type étant en nombre égal au nombre des cristaux de chaque détecteur élémentaire.

Selon d'autres modes de réalisation de l'invention:

—chaque photomultiplicateur du premier type est relié à chacun des cristaux d'un détecteur élémentaire par une liaison à fibre de verre,

—chaque photomultiplicateur d deuxième type est relié à un cristal de même rang de chaque détecteur élémentaire par une liaison à fibre de verre,

—tous les photomultiplicateurs sont reliés électriquement à un calculateur primaire qui délivre des signaux numériques d'énergie et de position de chaque photon détecté, au calculateur chargé de la reconstructions des images tomographiques,

—les photomultiplicateurs du premier type sont calibrés pour délivrer un signal proportionnel à l'énergie des photons émis par un cristal,

—les photomultiplicateurs du deuxième type sont prévus pour délivrer une impulsion permettant d'identifier le rang du cristal correspondant.

L'invention est décrite ci-après avec référence aux dessins annexés sur lesquels on peut voir:

Figure 1: une représentation schématique de la disposition des détecteurs élémantaires dans l'exemple d'une observation sous huit incidences au moyen d'un détecteur multiple selon l'invention;

Figure 2: un schéma symbolique de la disposition des appareils de mesure par rapport aux détecteurs élémentaires de la figure 1.

En se reportant au dessin, on voit que le détecteur multiple selon l'invention est composé d'un certain nombre de détecteurs élémentaires.

Dans l'observation d'un organe, un détecteur élémentaire est prévu pour chaque incidence et pour chaque coupe.

Dans l'exemple décrit, à huit incidences et cinq coupes, il y a donc quarante détecteurs élémentaires. Les détecteurs élémentiares de la première coupe sont numérotés de 1 à 8, ils correspondent chacun à une incidence.

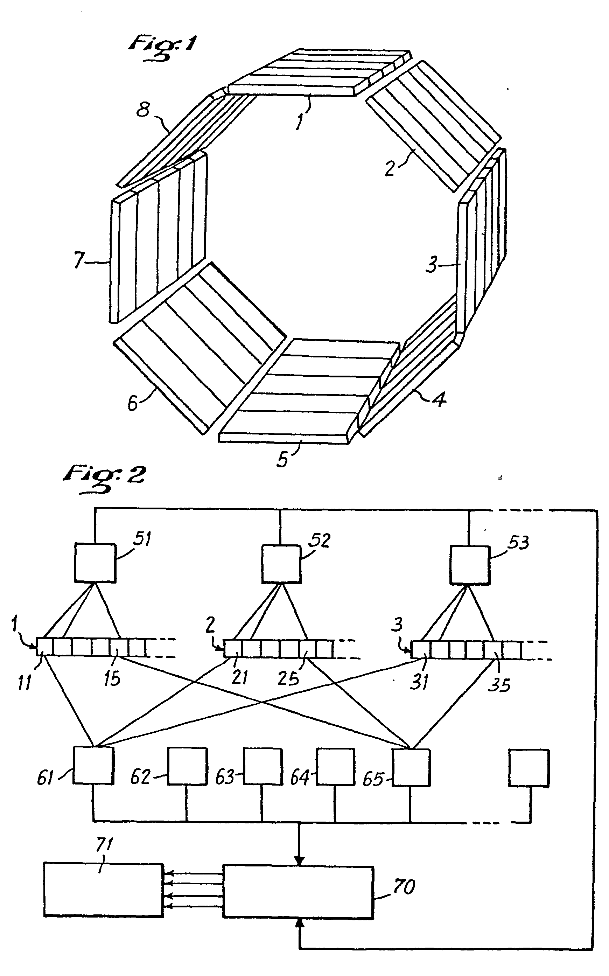

Un détecteur élémentaire se présente sous forme d'une barrette constituée d'un certain nombre de cristaux juxtaposés, trente-deux par exemple. Chacun de ces cristaux est par exemple un carré de 8 mm de côté. Les barrettes 1, 2 et 3 de la figure 2 correspondent aux détecteurs élémentaires 1, 2, 3 de la figure 1, et ont seules été représentées. Chaque cristal d'une barrette porte une référence correspondant à son rang. Ainsi, le cristal 15 est le cinquième cristal de la barrette 1, et le cristal 25 le cinquième cristal de la barrette 2, et ainsi de suite.

Selon l'invention, chaque cristal d'une barrette se trouve reliés à deux photomultiplicateurs de types différents: un photomultiplicateur de mesure d'énergie, tel que 51, 52, 53; et un photomultiplicateur de détermination du rang du cristal, tel que 61, ... 65.

La liaison entre cristal et photomultiplicateur est avantageusement réalisée par fibre de verre puisqu'il faut transmettre au photomultiplicateur l'énergie lumineuse émise par la scintillation du cristal sous l'effet des rayons gamma émis par l'organe à observer qui est placé au centre de la figure 1 de façon à pouvoir être vu simultanément sous les huit incidences prévues, et dans les cinq coupes prévues.

Ainsi, le photomultiplicateur d'énergie 51 reçoit les signaux lumineux de tous les cristaux de la barrette 1, et le photomultiplicateur 61, de détermination du rang du cristal, reçoit les signaux de tous les cristaux de rang 1. Dans l'exemple de réalisation proposé, où sont prévues huit incidences et cinq coupes, on a donc quarante barrettes telles que 1, 2 et 3. Chaque barrette comporte elle-même trente-deux cristaux. On a donc quarante photomultiplicateurs d'énergie tels que 51, et trente-deux photomultiplicateurs de détermination de rang tels que 61. L'ensemble des photomultiplicateurs est relié électriquement au calculateur primaire 70 qui a un rôle multiple. Tout d'abord il réalise une spectrométrie à partir des photomultiplicateurs d'énergie, pour ne prendre en compte que les photons dont l'énergie est située entre deux limites choisies par l'utilisateur en fonction du radio-élément utilisé, afin d'éliminer le rayonnement diffusé. Ensuite, il calcule, pour chaque photon retenu, la position du cristal ayant détecté ce photon, à partir des signaux des photomultiplicateurs d'énergie et des photomultiplicateurs de rang. Enfin, pour chaque photon détecté, il fournit sous forme numérique au calculateur 71 chargé de la reconstruction des coupes tomographiques, les quatre grandeurs: énergie, numéro de coupe, numéro d'incidence, et rang du cristal.

Lorsqu'un rayon gamme frappe un cristal tel que 25, c'est-à-dire le cristal de rang 5 de la barrette 2, ce cristal scintille en émettant un photon d'énergie déterminée. Le photomultiplicateur d'énergie 52 délivre un signal proportionnel à l'énergie du photon. Le photomultiplicateur 65 de détermination de rang délivre une impulsion permettant d'identifier le rang du cristal 25. La coïncidence du signal d'énergie et de l'impulsion de rang permet au calculateur primaire 70 de situer le cristal avec précision. Il faut remarquer que le signal d'énergie est proportionnel à l'énergie du photon.

Le calculateur primaire 70 délivre des signaux numériques d'énergie et de position de chaque photon détecté, au calculateur 71 chargé de la reconstruction des images tomographiques.

Selon l'invention, le détecteur multiple est constitué d'un certain nombre de détecteurs élémentaires ou barrettes, eux-mêmes constitués d'un certain nombre de cristaux juxtaposés. Ces détecteurs élémentaires étant disposés géométriquement autour de l'organe à observer pour le voir sous plusieurs incidences, on peut en une seule mesure obtenir toutes les incidences et toutes les coupes désirées. Ce temps d'immobilisation de l'appareil est cond considérablement plus court que celui d'une gamme-camera tournante.

Par ailleurs, le fait de prévoir deux groupes de photomultiplicateurs à fonctions différentes permet de réduire dans de fortes proportions le nombre des photomultiplicateurs tout en conservant une qualité de mesure remarquable.

S'il s'avère nécessaire d'augmenter la précision de mesure, on peut n'affecter les groupes de photomultiplicateurs d'énergie qu'à la moitié des cristaux des barrettes, c'est-à-dire en prévoir deux pour chaque barrette, et par ailleurs doubler le nombre des photomultiplicateurs de rang en ne les affectant chacun qu'à la moitié du nombre des barrettes. Ce doublement du nombre des photomultiplicateurs n'est nullement rédhibitoire.

Enfin un autre avantage du détecteur multiple selon l'invention est sa modularité. On peut en effet augmenter ou diminuer le nombre des détecteurs élémentaires en fonction de l'organe étudié (coeur, cerveau, foie) pour avoir le nombre souhaité de couples et d'incidences. Ce détecteur multiple et modulaire permet d'enregistrer simultanément toutes les incidences de toutes les coupes: il est particulièrement bien adapté à la tomoscintigraphie.

**Revendications**

1. Détecteur multiple pour tomoscintigraphie, du type à cristal sensible à une seule incidence, comportant des détecteurs élémentaires dont le nombre correspond au produit du nombre d'incidences par le nombre de coupes, pour recevoir simultanément toutes les incidences de toutes les coupes désirées, chaque détecteur élémentaire étant constitué par une barrette (1, 2 ...) comportant une certain nombre de cristaux juxtaposés, (11 ..., 15 ...; 21 ..., 25 ...) et orientée pour recevoir une incidence d'une coupe déterminée, chaque cristal d'une barrette étant relié à deux photomultiplicateurs, caractérisé en ce que lesdits photomultiplicateurs, caractérisé en ce que lesdits photomultiplicateurs sont de types différents, le premier est un photomultiplicateur d'énergie, (51, 52 ...) le second est un photomultiplicateur de détermination de rang, (61, 62 ...), les photomultiplicateurs du premier type étant en nombre égal au nombre de détecteurs élémentaires (1, 2 ...) et les photomultiplicateurs du second type étant en nombre égal au nombre des cristaux (11, ... 15 ...) de chaque détecteur élémentaire.

2. Détecteur selon la revendication 1, caractérisé en ce que chaque photomultiplicateur (51, ...) du premier type est relié à chacun des cristaux (11, ... 15, ...) d'un détecteur élémentaire (1, ...) par une liaison à fibre de verre.

3. Détecteur selon la revendication 1, caracaté- risé en ce que chaque photomultiplicateur (61, ...) du deuxième type est relié à un cristal (11, 21 ...) de même rang de chaque détecteur élémentaire (1, 2, ...) par une liaison à fibre de verre.

4. Détecteur selon la revendication 1, caractérisé en ce que tous les photomultiplicateurs (51, 52 ..., 61, ...) sont reliés électriquement à un calculateur primaire (70) qui délivre des signaux numériques d'énergie et de position de chaque photon détecté, au calculateur (71) chargé de la reconstruction des images tomographiques.

5. Détecteur selon la revendication 1, caractérisé en ce que les photomultiplicateurs (51, 52, ...) du premier type sont calibrés pour délivrer un signal proportionnel à l'énergie des photons émis par un cristal.

6. Détecteur selon la revendication 1, caractérisé en ce que les photomultiplicateurs (61, 62, ...) du deuxième type sont prévus pour délivrer une impulsion permettant d'identifier le rang du cristal correspondant.

## Patentansprüche

1. Vielfachdetektor für Tomoszintigraphie mit einem für einen einzigen Einfallwinkel empfindlichen Kristall, mit Einzeldetektoren deren Anzahl dem Produkt der Anzahl der Einfällen mit dem Anzahl der Schichten entspricht, um gleichzeitig alle Einfällen von allen erwünschten Schnitten zu empfangen, wobei jeder Einzeldetektor als Steg (1, 2 ...) ausgebildet ist, der eine gewisse Anzahl von nebeneinander angeordneten, um einen Einfall eines bestimmten Schnittes zu empfangen gerichtete Kristallen (11 ..., 15 ...; 21 ..., 25 ...) aufweist, wobei jeder Kristall eines Steges mit zwei Photomultipliers verbunden ist, dadurch gekennzeichnet, daß die Photomultipliers zu verschiedenen Gattungen gehören, wobei der Photomultiplier der ersten Gattung ein Energiemultiplier (51, 52 ...) und der Photomultiplier der zweiten Gattung ein Ordnungermittlungsmultiplier (61, 62 ...) ist, und daß die Anzahl der Photomultipliers der ersten Gattung der Anzahl der Einzelndetektoren (1, 2 ...) gleich ist und die Anzahl der Photomultipliers der zweiten Gattung der Anzahl der Kristallen (11, ... 15 ...) jedes Einzelndetektors gleich ist.

2. Vielfachdetektor nach Anspruch 1, dadurch gekennzeichnet, daß jeder Photomultiplier (51, ...) der ersten Gattung mit jedem Kristall (11, ... 15 ...) eines Einzelndetektors (1, ...) durch eine Glasfaserverbindung verbunden ist.

3. Vielfachdetektor nach Anspruch 1, dadurch gekennzeichnet, daß jeder Photomultiplier (61, ...) der zweiten Gattung mit einem Kristall gleicher Ordnung (11; 21 ...) jedes Einzelndetektors (1, 2 ...) durch eine Glasfaserverbindung verbunden ist.

4. Vielfachdetektor nach Anspruch 1, dadurch gekennzeichnet, daß alle Photomultipliers (51, 52 ..., 61, ...) elektrisch mit einem Primärrechner (70) verbunden sind, der digitale Signale, entsprechend der Energie und der Stelle jedes ermittelten Photons, an den mit der Wiedererstellung der tomographischen Bildern beauftragten Rechner (71) abgibt.

5. Vielfachdetektor nach Anspruch 1, dadurch gekennzeichnet, daß die Photomultipliers (51, 52, ...) der ersten Gattung zur Aufgabe eines zur Energie des von einem, Kristall gesendeten Photons poportionalen Signals geeicht sind.

6. Vielfachdetektor nach Anspruch 1, dadurch gekennzeichnet, daß die Photomultipliers (61, 62 ...) der zweiten Gattung zur Aufgabe eines die Feststellung der Ordnung des entsprechenden Kristalls erlaubenden Impuls vorgesehen sind.

## Claims

1. A multiple detector for tomoscintigraphy, of the type where each crystal is sensitive to one single incidence, comprising elementary detectors the number of which corresponds to the number of incidences multiplied by the number of sections for receiving simultaneously all the incidences from all wanted sections, each elementary detector being formed by a block, (1, 2, ...) comprising a certain number of juxtaposed crystals (11, ... 15; ... 21, ... 25), their block being directed as to receive an incidence from a determined section, each crystal of a block being connected to two photomultipliers, characterized in that said photomultipliers are of different types, the first being an energy photomultiplier (51, 52, ...) and the second a row determination photomultiplier (61, 62 ...), the number of photomultipliers of the first type being equal to the number of elementary detectors (1, 2 ...) and the number of the photomultipliers of the second type being equal to the number of crystals (11, ... 15, ...) of each elementary detector.

2. A detector as claimed in claim 1, characterized in that each photomultiplier (51, ...) of the first type is connected to each crystal (11, ... 15, ...) of an elementary detector (1, ...) by means of a glass fiber connection.

3. A detector as claimed in claim 1, characterized in that each photomultiplier (61, ...) of the second type is connected to a crystal in the same row (11, 21 ...) of each elementary detector (1, 2, ...) by means of a glass fiber connection.

4. A detector as claimed in claim 1, characterized in that all photomultipliers (51, 52, ... 61, ...) are electrically connected to a primary computer (70) which supplies digital energy and position signals of each detected photon to the computer (71) responsible for the reconstruction of the tomographic images.

5. A detector as claimed in claim 1, characterized in that the photomultipliers (51, 52 ...) of the first type of calibrated to supply a signal proportional to the energy of the photons emitted by a crystal.

6. A detector as claimed in claim 1, characterized in that the photomultipliers 61, 62, ... of the second type of provided for supplying a pulse allowing the identification of the row of the corresponding crystal.

Fig. 1

Fig. 2